# EUROPEAN PATENT SPECIFICATION

(11) **EP 1 792 597 B1**
(45) Date of publication and mention of the grant of the patent: **01.07.2015**
(21) Application number: 05741594.5
(22) Date of filing: 23.05.2005
(51) Int. Cl.: A61H 3/00

(54) **CONTROLLER OF WALKING ASSISTING DEVICE**
KONTROLLVORRICHTUNG FÜR EINE GEHHILFE
MODULE DE COMMANDE DE DISPOSITIF D'AIDE A LA MARCHE

(30) Priority: 27.08.2004 JP 2004248167
(43) Date of publication of application: 06.06.2007
(73) Proprietor: HONDA MOTOR CO., LTD., Tokyo 107-8556 (JP)
(72) Inventor: ASHIHARA, Jun, c/o Kabushiki Kaisha Honda Gijutsu Kenkyusho, Wako-shi, Saitama 351-0193 (JP)
(74) Representative: Hague, Alison Jane
(86) International application number: PCT/JP2005/009339
(87) International publication number: WO 2006/022057

(56) References cited:
- EP-A1- 1 442 703
- EP-A1- 1 661 543
- WO-A1-03/106112
- JP-A- 07 163 607
- JP-A- 59 222 152
- JP-B1- 50 034 355
- US-A- 3 358 678

## Description

### TECHNICAL FIELD

The present invention relates to a control device for a walking assistance device that is incorporated with a power actuator in at least one of the hip joint and knee joint of the wearer for the purpose of providing an assisting force to the movement of the leg.

### BACKGROUND OF THE INVENTION

Various muscle assistance devices have been proposed as can be found in Japanese patent laid open publication No. 2003-250844, for instance, for the purpose of providing an assisting force to the movement of the leg by using a torque actuator consisting of an electric motor or the like attached to a side part of the hip or knee joint. For instance, such a muscle assistance device enables a person caring for a bedridden person to produce a force of the leg which is substantially greater than that the person is normally capable of producing or a person having a walking impediment owing to aging or the like to walk on his or her feet.

In the power assist device disclosed in the mentioned Japanese patent application, the movement of the leg of the user is detected by using a sensor provided between a fastener such as a belt for securing an exoskeletal beam to the thigh and the front surface of the thigh and other sensors that are provided in the toe and ankle. The drive force of the torque actuator is controlled according to the output electric signals of these sensors so as to assist the flexing movement of the leg.

WO 03/106112 and US 3358678 disclose the features of the precharacterising portion of claim 1.

### BRIEF SUMMARY OF THE INVENTION

However, it should be noted that a relatively quick stepping movement is required to be effected for a walking assistance device to effectively assist the reflexive movement of the leg when the wearer has tripped over something and makes an effort not to fall down. According to the conventional walking assistance device that causes the actuator to produce a movement assist force for the leg of the user by using a feedback control, if the electric motor and power circuit are designed so as to be able to achieve such a quick response, the electric motor and power circuit would become unacceptably large, and the weight and cost of the device would be similarly unacceptable. Also, the reflex reaction of the user may also be impeded for various reasons so that simply feeding back the force of the wearer to produce an amplified drive command and forward the same to the actuator would not be adequate for the walking assistance device to successfully prevent the wearer from falling down.

The present invention was made in view of such problems of the prior art, and a primary object thereof is to provide a control device for a walking assistance device that can operate appropriately in dealing with a reflexive action of the user in an unexpected situation such as when tripping over something.

In one aspect the invention provides a control device for a walking assistance device, the walking assistance device comprising an actuator provided in association with at least one of a hip joint and a knee joint of a user, comprising: a controller that computes an assist force for the corresponding joint to reduce an effort to flex the corresponding joint and controls the corresponding actuator accordingly; a sensor for detecting a walking state of the user; a determination unit for detecting an abnormal state of walking according to an output signal from the sensor; and a switching unit for invoking an abnormal mode when an abnormal state of walking is detected by the determination unit whereby the controller controls the actuator in a way different from that under a normal mode, the abnormal mode including a forced tripping evasion mode wherein the controller urges a walking attitude of the user toward an upright attitude by using the actuator; characterised in that the sensor is attached to an abdomen of the user to measure a tension of a muscle, and the determination unit comprises a differentiating circuit for differentiating an output of the sensor and a comparator circuit for comparing an output of the differentiating circuit with a predetermined threshold value.

Thus, by predefining an abnormal mode as an action appropriate for optimally regaining the attitude of the user when the attitude of the user has destabilized by the user tripping over something, it becomes possible to prevent the user from falling down with a minimum power requirement by invoking the abnormal mode when it is predicted that the attitude of the user is about to become abnormal. Thereby, the action to set the abnormal walking attitude to an upright attitude can be automated, and the burden on the user who may have an impeded reflex function may be reduced.

According to the first aspect of the present invention, the sensor is attached to a human body part to measure a tension of a muscle, and the determination unit comprises a differentiating circuit for differentiating an output of the sensor and a comparator circuit for comparing an output of the differentiating circuit with a predetermined threshold value.

Thereby, when the user has stumbled upon something, and made an effort to stabilize the user's attitude, as the extent of destabilization of the user can be detected as the extent of the contraction of the muscles, it is possible to detect the extent of destabilization in such a way as to correspond to the human sense. Furthermore, because the reaction of the muscles is directly detected, it is possible to detect significant changes in the attitude before they actually occur, and the time lag is significantly less than is the case when an acceleration sensor or gyro sensor is used. Also, as in preferred embodiments the instrumentation simply consists of applying a myoelectric sensor to a body part such as abdomen, the device can be constructed as a light-weight and compact device. As an additional advantage, no complex mechanical computations are required.

### BRIEF DESCRIPTION OF THE DRAWINGS

Now the present invention is described in the following with reference to the appended drawings, in which:
Figure 1 is an overall perspective view of the walking assistance device embodying the present invention;
Figure 2 is a side view showing the walking assistance device as worn by a person;
Figure 3 is a plan view of the pelvis support member;
Figure 4 is a back view of an essential part of the walking assistance device;
Figure 5 is a perspective view of the axial force sensor; and
Figure 6 is a flowchart showing the control flow that switches over between different control modes.

### DETAILED DESCRIPTION OF THE PREFERRED EMBODIMENTS

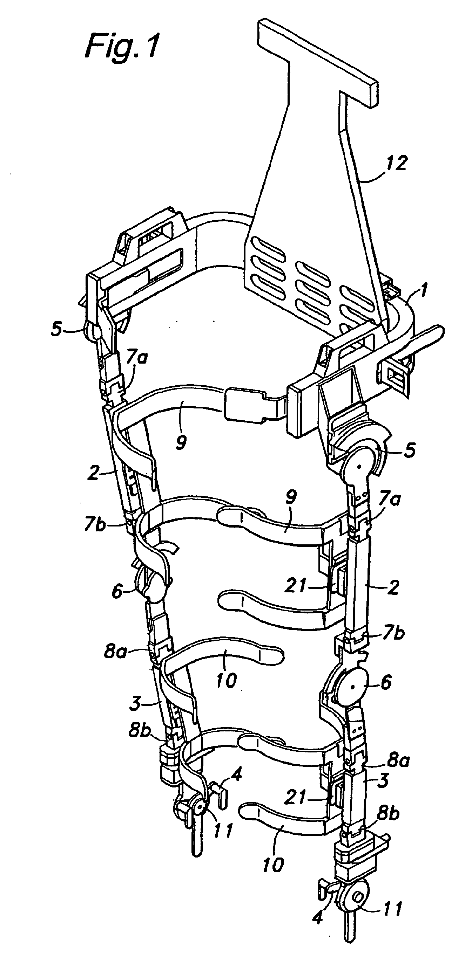

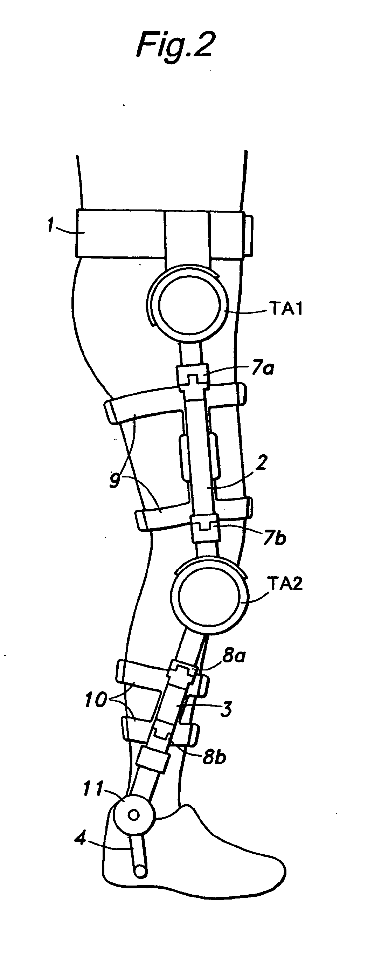

Figure 1 is an overall perspective view of an exoskeletal walking assistance device embodying the present invention, and Figure 2 is a side view showing the walking assistance device when worn by a user. As shown in Figure 1, the walking assistance device comprises a pelvis support member 1 adapted to be worn on the pelvis of the user, a thigh support member 2 which is vertically elongated so as to be placed on the outer side part of each thigh of the user, a leg support member 3 adapted to be placed on the outer part of each leg of the user, and a foot support member 4 adapted to be engaged by the shoe worn on each foot of the user. The "thigh" as used herein means a part of the limb extending between the hip joint and knee joint, and the "leg" as used herein means a part of the limb extending between the knee joint and ankle.

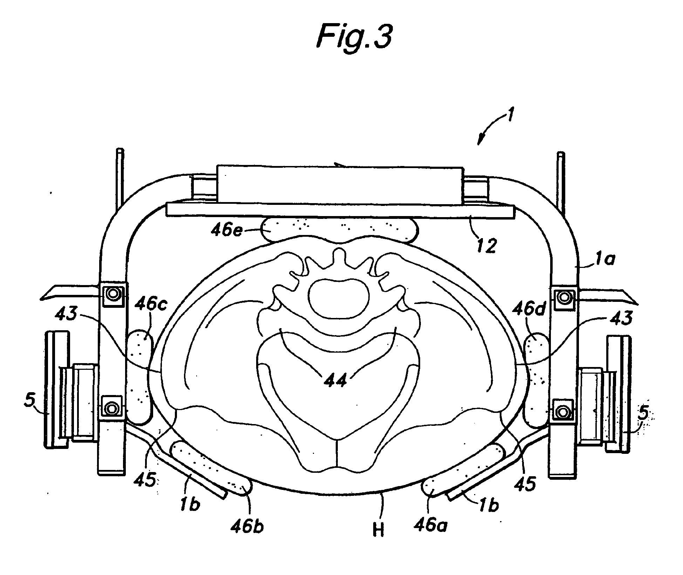

As shown in Figure 3, the pelvis support member 1 includes a base portion 1a made of a relatively rigid material and provided with the shape of letter-U as seen in plan view and a pair of flaps 1b each extending from a corresponding open front end of the base portion 1a and made of a resilient thin plate member so as to present the shape of letter-C including an open central front part. A middle part of the base portion 1a is adapted to be extended and retracted laterally while the flaps 1b are adapted to be extended and retracted in the fore-and-aft direction so as to accommodate the variable build of the wearer. The adjusted state of the base portion 1a can be immovably fixed by using suitable securing means such as threaded bolts.

The front end of each side portion of the pelvis support member 1 is provided with a pelvis actuator base 5 for mounting a first torque actuator TA1 (Figure 2) for applying a torque to the pelvic joint of the corresponding side. The output end of the first torque actuator TA1 is connected to the upper end of the thigh support member 2. The lower end of each thigh support member 2 is provided with a knee actuator base 6 for mounting a second torque actuator TA2 (Figure 2) for applying a torque to the knee joint of the corresponding side. The output end of the second torque actuator TA2 is connected to the upper end of the leg support member 3.

The thigh support member 2 is joined to the output end of the first torque actuator TA1 and knee actuator base 6, in each case, in an articulated manner via a hinge 7a, 7b having a hinge axis extending in the fore-and-aft direction. The leg support member 3 is joined to the output end of the second torque actuator TA2 and foot support member 4, in each case, in an articulated manner via a hinge 8a, 8b having a hinge axis extending in the fore-and-aft direction. The thigh support member 2 and leg support member 3 are each adapted to be slidably extended and retracted so as to accommodate the variable stature of the wearer. The adjusted lengths of the thigh support member 2 and leg support member 3 can be immovably fixed by using suitable securing means such as threaded bolts.

Each of the torque actuators TA1, TA2 consists of an electric motor fitted with a clutch and a reduction gear, and the motor housings thereof are mounted on the actuator bases 5,6 provided on the pelvis support member 1 and the lower end of the thigh support member 2, respectively, while the output ends or rotors thereof are fixedly connected to the upper ends of the thigh support member 2 and leg support member 3, respectively. Thereby, an assisting torque corresponding to the intended movement of each of the hip joint (the joint between the pelvis support member land thigh support member 2) and knee joint (the joint between the thigh support member 2 and leg support member 3) is produced. The torque actuators TA1, TA2 may be attached to the corresponding actuator bases 5,6 by using fastening means that can be repeatedly fastened and unfastened so that the maintenance and repair of the torque actuators may be facilitated.

Each thigh support member 2 is provided with a pair of cross members 9 each made of a C-shaped resilient member in a vertically spaced manner. The cross members 9 are jointly mounted at a suitable point on the thigh support member 2 in a vertically slidable manner, and adapted to attach the thigh support member 2 to the thigh of the wearer. Each leg support member 3 is similarly provided with a pair of cross members 10 each made of a C-shaped resilient member in a vertically spaced manner. The cross members 10 are jointly mounted at a suitable point on the leg support member 3 in a vertically slidable manner, and are adapted to attach the leg support member 3 to the leg of the wearer.

The joint between each leg support member 3 and corresponding foot support member 4 is rotatable around a laterally extending axis so as to accommodate the normal movement of the ankle.

To a central part of the pelvis support member 1 is attached a back plate 12 for mounting auxiliary equipment not shown in the drawings. For instance, a control circuit and a battery are mounted on the back plate 12.

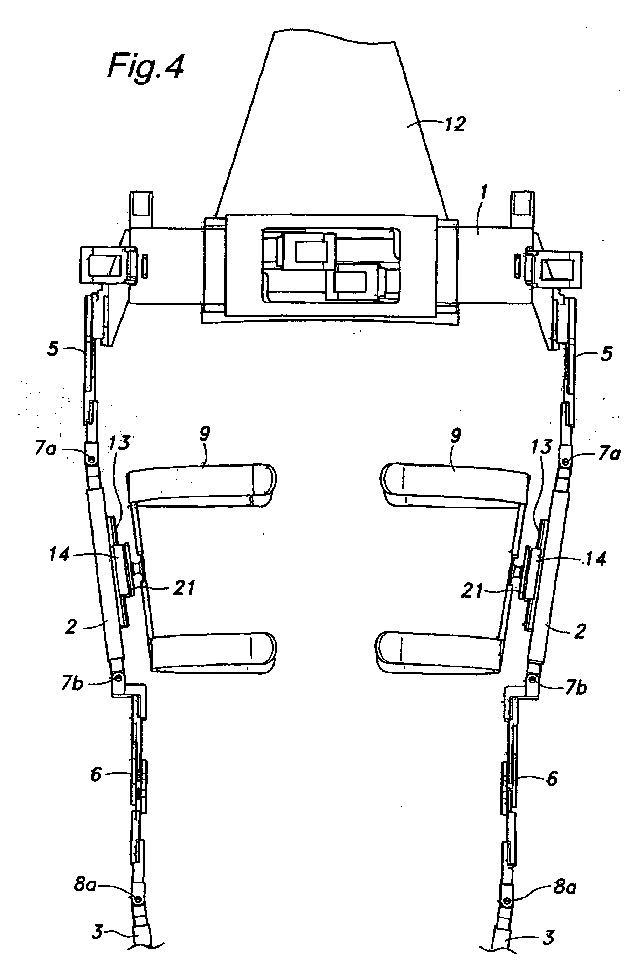

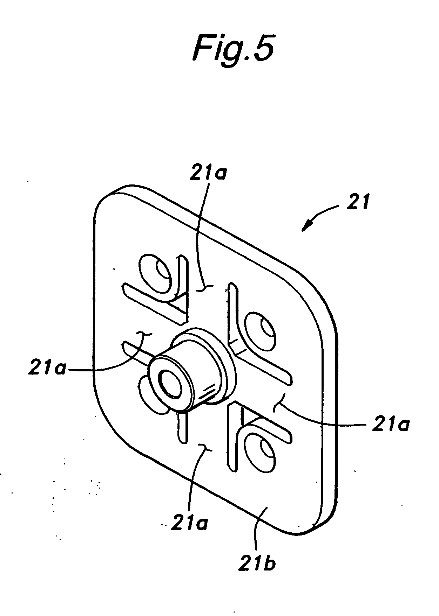

As additionally shown in Figure 4, an axial force sensor 21 is provided in each of the joined parts between the thigh support member 2 and corresponding cross members 9 and between the leg support member 3 and corresponding cross members 10. As shown in Figure 5, the axial force sensor 21 is made of a plate member formed with four L-shaped cutouts so as to define a cross shaped portion having four flexible arms 21a and a surrounding rectangular frame portion 21b. This may be formed by machining a plate member made of structural carbon steel, and a strain gage (not shown in the drawings) such as a wire resistance strain gage is attached to each of the flexible arms 21a of the cross shaped portion. The base end of each of the cross members is attached to the central part of the cross shaped portion at which the flexible arms 21a intersect with each other. The frame portion 21b is fixedly attached to a carriage 14 slidably engaging a guide rail 13 mounted on the thigh support member 2 or leg support member 3, as the case may be.

The axial force sensor 21 is capable of detecting a composite force that may include vertical, lateral and fore-and-aft components, and is therefore capable of detecting the force acting between the thigh support member 2 and base end of the cross members 9 or the force acting between the leg support member 3 and base end of the cross members 10 with respect to all directions.

For instance, when the thigh is swung forward around the hip joint, the cross members 9 of the thigh support member 2 receive a force that is directed forward, and this is detected by the axial force sensor 21. Then, the torque actuator TA1 provided on a side of the hip joint produces such a torque that would make the thigh support member 2 follow the movement of the thigh of the wearer. If the axial force sensor 21 detects a certain amount of force although the wearer is standing still, it means that the weight of the walking assistance device is acting upon the leg of the wearer. In such a case, the outputs of the two torque actuators TA1, TA2 may be feedback controlled so as to minimize the force detected by the axial force sensor 21. Thereby, most of the weight of the walking assistance device may be transmitted to the floor surface via the sole of the shoe so as to cancel it with the reaction from the surface, and the burden of the walking assistance device on the wearer can be minimized.

Normally, when a human tries to maintain an upright attitude, the muscles around the vertebrae are required to be strained. When the balance is lost, it is known that the ventrolateral oblique muscles of either side are instantaneously contracted so as to put the upper part of the human body back to the upright posture in an effort to maintain an upright attitude. By noting such an instinctive human reaction to maintain the upright posture and detecting the strain of the muscles around the abdomen, it is possible to detect an early indication that the walking posture may be about to be thrown off balance.

It is known to attach electrodes to the skin surface to detect the action potential of the muscles as a means for monitoring the strained state of the muscles. More specifically, a plurality of surface electrodes each measuring about 10 mm in diameter are attached to the body surface along the muscle fibers of the ventrolateral oblique muscles at the interval of about 15 mm to detect the action potential of the ventrolateral oblique muscles. The total number of the surface electrodes is selected appropriately.

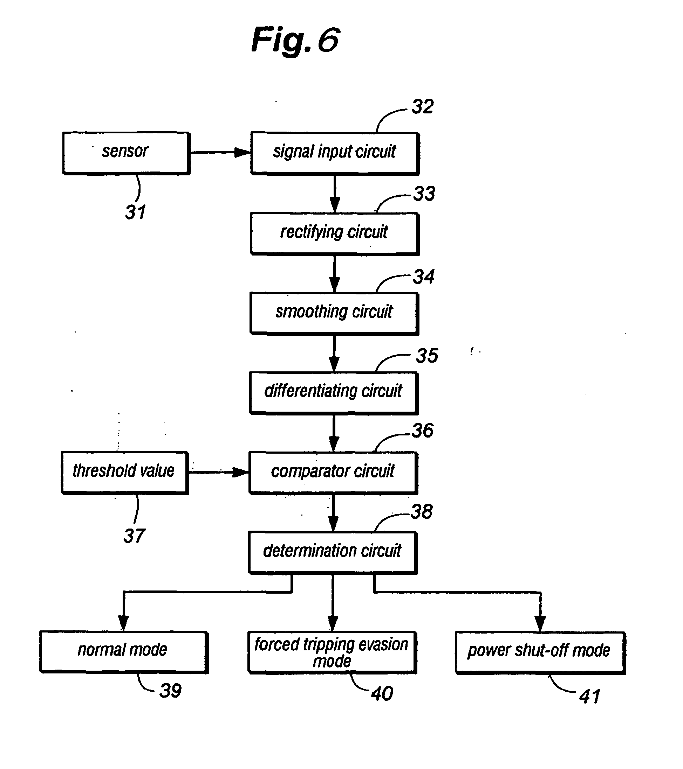

As shown in Figure 6, the myoelectric signal detected by the myoelectric sensor 31 is supplied to a signal input circuit 32, and full-wave rectified by a rectifying circuit 33. The signal is then smoothed by a smoothing circuit 34 that takes a moving average, for instance. The smoothed signal is differentiated by a differentiating circuit 35, and is forwarded to a comparator circuit 36 to be compared with a prescribed threshold valve 37. If the signal is below the threshold value 37, a determination circuit 38 issues a command to continue to produce the assist torque according to a feedback control based on the output of the axial force sensor 21 under a normal mode 39. If the signal is above the threshold value 37, the determination circuit 38 determines that the walking movement of the wearer is not normal, for instance by detecting that the wearer may be about to trip over his feet, and terminates the feedback control based on the axial force sensor 21. In this case, depending of the nature of the walking impediment of the wearer, the determination circuit 38 initiates a forced tripping evasion mode 40 or a power shut off mode 41.

In the forced tripping evasion mode 40, the changes in the attitude of the wearer are predicted according to the rotational angles of the torque actuators TA1, TA2 provided in the hip and knee joints and the pattern of the detected meyogenic signal mentioned above, and the torque actuators TA1, TA2 are automatically controlled according to the predetermined torque target values so that the most appropriate action may be taken by the legs involved in evading the tripping of the wearer. In the power shut-off mode 41, if it is determined that the assist torques produced from the torque actuators TA1, and TA2 would not be adequate for evading the tripping of the wearer, the wearer may be allowed to fall down by shutting off the transmission of power from the torque actuators TA1, TA2 to the thigh and leg support members 2, 3 for instance by disengaging the clutches incorporated in the torque actuators TA1, TA2.

For the protection of the wearer when the wearer is allowed to fall down in the power shut-off mode, as shown in Figure 3, cushioning pads 46c - 46e are provided on the U-shaped based portion 1a of the pelvis support member so as to oppose the part of the pelvic portion of the wearer H extending from the crest portions of the right and left iliac regions (the lateral side ends of the pelvis) 43 to the back side of the sacroiliac joint (joint between the vertebrae and pelvic bone) 44, and cushioning pads 46a, 46b are provided on the right and left flap portions 1b opposing the right and left spina iliac regions (front ends of the pelvic bone) 45. Thereby, the pelvis support member 1 serves as a protector that protects the pelvis H of the wearer from the damages of falling down.

As discussed in connection with the foregoing embodiment, if the determination process is based on the differentiated value or the change in a given time period of the myogenic potential, the determination process would respond only when a sudden change takes place, and would not respond when the change is highly gradual although the peak value of the myogenic potential may be great. In other words, the system would not unnecessarily respond when the change is so gradual that the attitude of the wearer can be regained even under the normal mode no matter how great the magnitude of the change in the attitude may be. As an additional advantage arising from this feature, an offset that may be present in the output of the sensor would not affect the operation of the system because the system does not respond to the absolute value (DC value) of the change.

Contraction of a muscle can be detected not only by the method mentioned above but also by a method using a piezoelectric sensor to measure the hardness of the muscle. These different methods of measuring the contraction of a muscle can be used individually or in combination. The present invention can be applied not only to such an exoskeltal walking assistance device as described above but to also to those that use shape memory alloy, high polymer or pneumatic devices for the actuators (see Japanese patent laid open publication No. 2002-48053 if necessary).

Although the present invention has been described in terms of preferred embodiments thereof, it is obvious to a person skilled in the art that various alterations and modifications are possible without departing from the scope of the present invention which is set forth in the appended claims.

## Claims

1. A control device for a walking assistance device, the walking assistance device comprising an actuator (TA1, TA2) provided in association with at least one of a hip joint and a knee joint of a user, comprising:
a controller that computes an assist force for the corresponding joint to reduce an effort to flex the corresponding joint and controls the corresponding actuator accordingly;
a sensor (31) for detecting a walking state of the user;
a determination unit (32-36) for detecting an abnormal state of walking according to an output signal from the sensor; and
a switching unit (38) for invoking an abnormal mode (40,41) when an abnormal state of walking is detected by the determination unit whereby the controller controls the actuator in a way different from that under a normal mode, the abnormal mode including a forced tripping evasion mode (40) wherein the controller urges a walking attitude of the user toward an upright attitude by using the actuator;
**characterised in that** the sensor (31) is attached to an abdomen of the user to measure a tension of a muscle, and the determination unit comprises a differentiating circuit (35) for differentiating an output of the sensor and a comparator circuit (36) for comparing an output of the differentiating circuit with a predetermined threshold value (37).

2. The control device according to claim 1, wherein the abnormal mode further includes a power shut off mode (41) whereby transmission of force from the actuator (TA1, TA2) is shut off, the power shut off mode being invoked instead of the forced tripping evasion mode (40), when the abnormal state of walking is detected and it is determined that the actuator is not able to provide an adequate force for evading the tripping of the user.

## Patentansprüche

1. Eine Steuervorrichtung für eine Gehhilfevorrichtung, die Gehhilfevorrichtung aufweisend einen Aktuator (TA1, TA2) bereitgestellt in Verbindung mit zumindest einem von einem Hüftgelenk und einem Kniegelenk von einem Anwender, aufweisend:
einen Steuerer, der eine Hilfskraft für das korrespondierende Gelenk berechnet, um eine Anstrengung beim Beugen des korrespondierenden Gelenks zu reduzieren, und den korrespondierenden Aktuator entsprechend steuert;
einen Sensor (31) zum Detektieren eines Gehzustands von dem Anwender;
eine Bestimmungseinheit (32 - 36) zum Detektieren eines krankhaften Gehzustands gemäß einem Ausgangssignal von dem Sensor; und
eine Umschalteinheit (38) zum Aufrufen eines Krankhaftmodus (40, 41), wenn ein krankhafter Gehzustand durch die Bestimmungseinheit detektiert wird, wobei der Steuerer den Aktuator auf eine Weise steuert, die sich von derjenigen unter einem Normalmodus unterscheidet, der Krankhaftmodus umfasst einen Zwangsstolpervermeidungsmodus (40), wobei der Steuerer eine Gehhaltung von dem Nutzer unter Verwendung des Aktuators hin zu einer aufrechten Haltung drängt;
**dadurch gekennzeichnet, dass** der Sensor (31) am Unterleib von dem Anwender befestigt ist, um eine Spannung von einem Muskel zu messen, und dass die Bestimmungseinheit eine Differenzierschaltung (35) zum Differenzieren einer Ausgabe von dem Sensor und eine Vergleichsschaltung (36) zum Vergleichen einer Ausgabe von der Differenzierschaltung mit einem vorherbestimmten Schwellenwert (37) aufweist.

2. Die Steuervorrichtung gemäß Anspruch 1, wobei der Krankhaftmodus weiter einen Kraftabschaltmodus (41) beinhaltet, wobei eine Übertragung von Kraft von dem Aktuator (TA1, TA2) abgeschaltet wird, der Kraftabschaltmodus wird anstatt des Zwangsstolpervermeidungsmodus (40) aufgerufen, wenn der krankhafte Gehzustand detektiert wird und bestimmt wird, dass der Aktuator nicht in der Lage ist, eine angemessene Kraft zum Vermeiden des Stolperns von dem Nutzer bereitzustellen.

## Revendications

1. Dispositif de commande pour un dispositif d'assistance à la marche, le dispositif d'assistance à la marche comprenant un actionneur (TA1, TA2), aménagé en association avec au moins l'une d'une articulation de hanche et d'une articulation de genou d'un utilisateur, comprenant :
un contrôleur qui calcule une force d'assistance pour l'articulation correspondante afin de réduire un effort pour fléchir l'articulation correspondante et commande l'actionneur correspondant en conséquence ;
un capteur (31) pour détecter un état de marche de l'utilisateur ;
une unité de détermination (32-36) pour détecter un état de marche anormal selon un signal de sortie du capteur ; et
une unité de commutation (38) pour invoquer un mode anormal (40, 41) lorsqu'un état de marche anormal est détecté par l'unité de détermination, de sorte que le contrôleur commande l'actionneur de manière différente de celle utilisée dans un mode normal, le mode anormal comprenant un mode de dérobade à un faux pas forcé (40), dans lequel le contrôleur contraint l'utilisateur à convertir son attitude de marche en une attitude droite en utilisant l'actionneur ;
**caractérisé en ce que** le capteur (31) est fixé à l'abdomen de l'utilisateur pour mesurer la tension d'un muscle et l'unité de détermination comprend un circuit de différenciation (35) pour différencier une sortie du capteur et un circuit comparateur (36) pour comparer une sortie du circuit de différenciation à une valeur de seuil prédéterminée (37).

2. Dispositif de commande selon la revendication 1, dans lequel le mode anormal comprend en outre un mode de coupure d'énergie (41) de sorte que la transmission de force de l'actionneur (TA1, TA2) soit coupée, le mode de coupure d'énergie étant invoqué au lieu du mode de dérobade à un faux pas forcé (40) lorsque le mode de marche anormal est détecté et que l'on détermine que l'actionneur n'est pas à même de fournir une force adéquate pour que l'utilisateur se dérobe au faux-pas.
